## Europäisches Patentamt

## European Patent Office

## Office européen des brevets

(11) Veröffentlichungsnummer: **0 221 880**
**B1**

(12) # EUROPÄISCHE PATENTSCHRIFT

(45) Veröffentlichungstag der Patentschrift:
02.08.89

(51) Int. Cl.⁴: **C 07 C 59/265,** C 07 C 51/41

(21) Anmeldenummer: **86890267.7**

(22) Anmeldetag: **25.09.86**

(54) **Verfahren zur Isolierung von hochreinem Tricalciumcitrat aus citronensäurehältigen wässerigen Lösungen.**

(30) Priorität: **03.10.85 AT 2864/85**

(43) Veröffentlichungstag der Anmeldung:
**13.05.87 Patentblatt 87/20**

(45) Bekanntmachung des Hinweises auf die Patenterteilung:
**02.08.89 Patentblatt 89/31**

(84) Benannte Vertragsstaaten:
**DE FR GB IT NL SE**

(56) Entgegenhaltungen:
**US-A-2 027 264**
**US-A-2 072 530**
**US-E-28 192**

(73) Patentinhaber: **VOGELBUSCH GESELLSCHAFT m.b.H., Blechturmgasse 11, A-1050 Wien (AT)**

(72) Erfinder: **Salzbrunn, Wolfgang, Dr., Bräunlichgasse 11, A-2700 Wr. Neustadt (AT)**
Erfinder: **Kominek, Jiri, Dipl.- Ing., Zeltgasse 2/1/4, A-1080 Wien (AT)**
Erfinder: **Röhr, Max, Prof., Asenbauergasse 32- 34, A-1230 Wien (AT)**

(74) Vertreter: **Wolfram, Gustav, Dipl.- Ing., Schwindgasse 7 P.O. Box 205, A-1041 Wien (AT)**

## Beschreibung

Die Erfindung betrifft ein Verfahren zur Isolierung von hochreinem Tricalciumcitrat-Tetrahydrat durch Zusatz eines calciumhältigen Fällungsmittels zu citronensäurehältigen wässerigen Lösungen bei erhöhter Temperatur.

Calciumcitrat-Fällungsverfahren sind insbesondere zur Gewinnung von Citronensäure aus Fermentationsflüssigkeiten geeignet, doch kann auch beispielsweise die im Saft von Citrusfrüchten enthaltene Citronensäure auf ähnliche Weise gewonnen werden (siehe Ullmanns Encyklopädie der technischen Chemie, 3. Auflage, 5. Band, Seiten 606 bis 608, Verlag Urban 8 Schwarzenberg, 1954)

Die US-A-2 072 530 beschreibt die Gewinnung von Calciumcitrat aus geschälten und zerkleinerten Zitrusfrüchten. In einem ersten Ausfällungsschritt wird dabei ein kolloidaler Rückstand nicht näher definierter Zusammensetzung erhalten, der einen hohen Anteil an freier Citronensäure enthält. Um den Hauptanteil der Citronensäure zu gewinnen, muß der klare Überstand zunächst vom Rückstand dekantiert, dann der Rückstand ausgepreßt und aus dem Überstand und dem Preßsaft in der Folge mit Kalk die Citronensäure ausgefällt werden.

Die zu den wichtigsten Säuren in der Lebensmittelchemie zählende Citronensäure wird heute zum weitaus überwiegenden Teil durch Gärung mittels bestimmter Schimmelpilze - beispielsweise Aspergillus niger - hergestellt, wofür man zuckerhaltige Ausgangs- bzw. Rohstoffe, wie Melassen und Stärkehydrolysate oder auch reine Zucker selbst einsetzt. Dabei entstehen mehr oder weniger verdünnte Lösungen von Citronensäure oder von deren Alkalimetallsalzen, die außer dieser Säure noch weitere Substanzen enthalten. Diese Begleitsubstanzen liegen, besonders bei Vergärung von Melassen, in etwa gleicher Menge wie die Citronensäure selbst in den Fermentationslösungen vor. Aus diesen Lösungen muß die Citronensäure isoliert werden. Dazu hat sich die Fällung derselben als schwer lösliches Tricalciumcitrat als am besten geeignet erwiesen, wofür üblicherweise Kalkmilch als Fällungsmittel eingesetzt wird. Diese Fällung stellt nicht nur eine Isolierungsmethode dar, sondern ist auch jener Verfahrensschritt, bei dem die beste Gelegenheit besteht, die Citronensäure von den begleitenden Substanzen zu befreien.

Es ist bekannt, daß der Erfolg der Fällung von folgenden Parametern abhängt: Temperatur und pH-Wert zu Fällungsende, Verweilzeit des Calciumcitrates in der Suspension nach erfolgter Fällmittelzugabe sowie Qualität des Fällmittels selbst. Wie man weiters weiß, ist eine möglichst hohe Endtemperatur von etwa 70 bis 90°C anzustreben, da die Löslichkeit des Tricalciumcitrates mit steigender Temperatur sinkt. Für den pH-Wert gilt, daß mit steigenden Werten die Verluste abnehmen, allerdings nimmt zwischen pH 7 und 9 das gefällte Tricalciumcitrat erhebliche Mengen an Verunreinigungen aus der Gärlösung auf, so daß ein Kompromiß zwischen der Reinheit des Produktes und den Verlusten getroffen werden muß.

Aber selbst bei entsprechend diesen Erkenntnissen eingestellten niedrigeren pH-Werten ist das entstandene Tricalciumcitrat noch immer stark verunreinigt, weil Tricalciumcitrat unter den in der Praxis herrschenden Bedingungen immer quasiamorph in Form kleinster Partikel ausfällt, welche rasch zu Verbänden agglomerieren.

Die citronensäurehaltigen Lösungen neigen extrem stark zur Übersättigung an entstandenem Tricalciumcitrat, welches Phänomen den komplexierenden Eigenschaften der Citronensäure zuzuschreiben ist (Heinz, E.: Untersuchungen über Komplexverbindungen des Calciums, Biochem. Z.321, 319, 1951). Das Calciumsalz fällt bei fortgesetztem Fällungsmittelzusatz plötzlich aus, der aus Agglomeraten feinster Teilchen bestehende und daher oberflächenreiche Niederschlag ist zwar gut filtrierbar, doch muß ein starker Verunreinigungsgrad durch mitgefällte Begleitsubstanzen in Kauf genommen werden. Die Begleitsubstanzen sind selbst durch intensives Waschen nicht zu entfernen, weswegen aufwendige und kostspielige Reinigungsschritte im Zuge der weiteren Aufarbeitung notwendig sind.

Aus der DE-A-3 014 503 ist ein kontinuierliches Verfahren bekannt, wobei zur Verminderung der Mitfällung und des Einschlusses von Begleitstoffen die Vermischung der Lösung mit dem Fällungsmittel einerseits und die Reaktion der Komponenten, gegebenenfalls zusammen mit der Fällung, anderseits räumlich und zeitlich voneinander getrennt durchgeführt wird.

Diese Vorgangsweise hat den Zweck, eine möglichst rasche Durchmischung der Komponenten in maximal 1 s zu erreichen, um örtliche Konzentrationsunterschiede zu vermeiden und plötzliches Ausfallen von Citrat zu verhindern. Das Verfahren nach der DE-A-3 014 503 führt zwar zu einem an Begleitstoffen ärmeren Produkt, ein deutlich kristallines und infolgedessen hochreines Calciumcitrat wird jedoch nicht erhalten. Dazu kommt noch, daß zur Durchführung des bekannten Verfahrens wenigstens ein Mischer und ein Rührkessel - vorzugsweise zusätzlich ein Strömungsrohr als Reaktionsgefäß - benötigt werden. Da die im wesentlichen drei Verfahrensstufen, nämlich Vermischung, Reaktion und Fällung, bei jeweils unterschiedlichen Temperaturen durchgeführt werden, sind für jede Stufe separate Heizungen sowie aufwendige meß- und regeltechnische Einrichtungen erforderlich. Ein vorzeitiger Ausfällungsbeginn kann selbst mit komplizierten Überwachungsvorrichtungen trotzdem nicht völlig ausgeschlossen werden.

Die Erfindung stellt sich die Aufgabe, die aufgezeigten Schwierigkeiten und Nachteile zu überwinden und ein vergleichsweise einfaches und betriebssicheres Verfahren zu schaffen, bei dem auch mit nur einem Reaktionsgefäß das Auslangen gefunden werden kann und hochreines - weil kristallines - Tricalciumcitrat-Tetrahydrat erhalten wird.

Die gestellte Aufgabe wird bei einem Verfahren der eingangs definierten Art erfindungsgemäß dadurch gelöst, daß nach Zugabe von etwa 25 bis 30 % der zur Bildung von Tricalciumcitrat benötigten, stöchiometrisch errechneten Menge an calciumhältigem Fällungsmittel zu der wässerigen Lösung die weitere Zugabe mit verminderter Geschwindigkeit bis zum ersten Auftreten einer Trübung durch kristallin ausfallendes Tricalciumcitrat-Tetrahydrat fortgesetzt, dann so lange unterbrochen wird, bis der Kristallisationsvorgang im wesentlichen beendet ist und nach Ablauf dieser Zeitspanne die restliche Menge des calciumhältigen Fällungsmittels zugegeben wird.

Die citronensäurehaltige wässerige Lösung muß nur einmal auf die Fällungstemperatur erhitzt werden, wobei eine hohe Aufheizgeschwindigkeit eingehalten werden kann. Nach Erreichen dieser Temperatur kann unmittelbar mit dem Zusatz des calciumhaltigen Fällungsmittels, wie Ca(OH)$_2$ (Kalkmilch), CaO oder CaCO$_3$ begonnen werden. Liegen die wässerigen Lösungen als annähernd neutrale Fermentationsflüssigkeit vor, kommen auch beispielsweise Calciumchlorid oder -sulfat als Fällungsmittel in Frage.

Die Überwachung des ersten Auftretens einer Trübung kann leicht automatisiert werden und die Steuerung der Zugabe von Fällungsmittel ist regeltechnisch sehr einfach zu lösen.

Betrachtet man die wässerige Lösung zum Zeitpunkt des ersten Auftretens einer Trübung unter dem Mikroskop, so erkennt man zunächst kleine Verbände von wohl ausgebildeten Calciumcitratkristallen. Während des Kristallwachstums werden auch diese Verbände laufend größer, indem sich weitere Kristalle anlagern. Dieses Entstehen neuer Kristalle und das Kristallwachstum finden statt, obwohl kein weiteres Fällungsmittel zugeführt wird. Wenn der Kristallisationsvorgang im wesentlichen beendet ist, d.h. wenn kein weiteres Anwachsen der Kristallverbände mehr beobachtet werden kann, wird die Zufuhr des calciumhältigen Fällungsmittels wieder aufgenommen und die Fällung ohne weitere Unterbrechung zu Ende geführt. Auch das während und nach der Zugabe der restlichen Menge des Fällungsmittels entstehende Calciumcitrat ist deutlich kristallin und das gesamte gefällte Tricalciumcitrat-Tetrahydrat ist einheitlich.

Dieses kristalline · Produkt ist ausgezeichnet waschbar.

Vorzugsweise wird die Zugabe der ersten etwa 25 bis 30 % sowie der restlichen Menge an calciumhältigem Fällungsmittel unter intensiver Durchmischung der Lösung mit hoher Geschwindigkeit - jedoch ohne lokale Überkonzentration von calciumhältigem Fällungsmittel in der Lösung - vorgenommen.

Solche örtlichen Überkonzentrationen bzw. Konzentrationsgradienten müssen auf jeden Fall vermieden werden, da diese das unerwünschte plötzliche Ausfällen des Calciumcitrates in amorpher Form bewirken würden. Es muß somit dafür gesorgt werden, daß das Fällungsmittel unmittelbar nach dem Einbringen in der Lösung möglichst homogen verteilt wird. Zum Durchmischen können irgendwelche bekannten Rührorganen wie Propellerrührwerke, verwendet werden, wobei aber darauf Bedacht zu nehmen ist, daß nicht durch Anwendung zu hoher Scherkräfte beim Durchmischen die Kristallisation behindert oder gebildete Kristalle mechanisch wieder zerstört werden.

Nach einer besonders bevorzugten Ausführungsform wird die Lösung während der Zugabe des calciumhältigen Fällungsmittels mit einem eine Hohlwelle aufweisenden Vibrationsmischer intensiv durchmischt, wobei das calciumhältige Fällungsmittel durch die Hohlwelle zugegeben wird.

Die Hohlwelle eines solchen Vibrationsmischers ist mit einem Schwingungserzeuger verbunden und weist an ihrem anderen Ende eine mit Öffnungen versehene Mischplatte auf (z. B. ähnlich dem Vibromischer mit Vollwelle in Ullmanns Encyklopädie der technischen Chemie, 3. Auflage, 1. Band, Seiten 702 und 703, Verlag Urban & Schwarzenberg 1951).

Ein Vibrationsmischer kann mit verschiedenen Schwingungsfrequenzen, beispielsweise mit der Netzfrequenz von etwa 50 Hz, betrieben werden und bewirkt eine sehr intensive Durchmischung der Flüssigkeit bei vergleichsweise geringem Energieaufwand. Das durch die Hohlwelle zugegebene Fällungsmittel tritt direkt unter der Mischplatte aus und wird in der wässerigen Lösung sofort feinst verteilt. In der Lösung können sich auf diese Weise nur äußerst kurz dauernde Konzentrationsgradienten in kleinsten Bereichen ausbilden.

Bei der zuletzt beschriebenen Ausführungsform wird zweckmäßig als calciumhältiges Fällungsmittel Calciumcarbonat zugegeben.

Calciumcarbonat ist erheblich billiger als das daraus durch Brennen gewonnene Calciumoxid, es bewirkt jedoch infolge CO$_2$-Entwicklung sehr starke Schaumbildung bei der Zugabe. Bei üblichem Rühren entweicht das in Form kleinster Bläschen in der Lösung vorliegende CO$_2$ zu langsam, sodaß das Schäumen nicht beherrscht werden kann. Bei Verwendung eines Vibrationsmischers findet als zusätzlicher Effekt eine derart rasche Koaleszenz der Bläschen statt, daß das entwickelte Gas viel schneller aus der Flüssigkeit entweichen kann. Der Vibrationsmischer wirkt somit ähnlich einem Ultraschallgenerator mit sehr geringer Energiedichte. Analog zu einem solchen Generator wird die Schaumbildung sofort unterdrückt und der Einsatz von Calciumcarbonat als Fällungsmittel ist ohne Schwierigkeiten möglich.

Zweckmäßig erfolgt die Zugabe des calciumhältigen Fällungsmittels ab einem pH-Wert der wässerigen Lösung von 3,0 mit verminderter Geschwindigkeit. Dieser pH-Wert wird in sauren Lösungen nach Zugabe von etwa 25 bis 30 % eines basischen Fällungsmittels erreicht; die Orientierung am pH-Wert ermöglicht eine einfache Steuerung der Fällungsmittelzugabe.

Die Zeitspanne, während der die Zugabe des Fällungsmittels unterbrochen werden muß, ist unter ähnlichen Fabrikationsbedingungen reproduzierbar und muß daher nicht bei jedem Verfahrensdurchgang neu ermittelt werden. Sie läßt sich als Erfahrungswert übernehmen.

Zweckmäßig wird die Zugabe calciumhältigen Fällungsmittels beim ersten Auftreten einer Trübung 1 bis 30 min lang, vorzugsweise 5 bis 10 min lang, unterbrochen. Zeitspannen innerhalb dieser Grenzen haben sich in der Praxis als geeignet erwiesen und sind für alle speziellen Randbedingungen der Verfahrensdurchführung anwendbar.

Es ist beim Ausgehen von sauren wässerigen Lösungen weiters von Vorteil, daß von der restlichen Menge des calciumhältigen Fällungsmittels so viel zugesetzt wird, bis sich in der wässerigen Lösung ein pH-wert von 5,5 einstellt. Wird mehr basisches Fällungsmittel zugesetzt, muß bei höheren pH-Werten mit einer gewissen Verunreinigung des kristallinen Calciumcitrates durch aus der Lösung mitgerissene Begleitstoffe gerechnet werden.

Als günstig hat sich auch herausgestellt, mit der Zugabe der ersten etwa 25 bis 30 % der Gesamtmenge an calciumhältigem Fällungsmittel bei einer Temperatur der wässerigen Lösung von 60 bis 90°C zu beginnen und die Temperatur der Lösung bei Zugabeende der restlichen Menge an calciumhältigem Fällungsmittel auf etwa 90°C bis 100°C einzustellen.

Infolge der freigesetzten Reaktionswärme braucht bei einer solchen Temperaturführung der wässerigen Lösung während der Durchführung des erfindungsgemäßen Verfahrens weitere Energie - wenn überhaupt - nur in sehr geringem Ausmaß zugeführt werden. In den meisten Fällen genügt es, das Entweichen von zu viel Wärme aus dem Reaktionsgefäß durch entsprechende Isolierung zu verhindern.

Aufgrund von Vorversuchen ist es leicht möglich, für ein bestimmtes Reaktionsgefäß durch Variation der Temperatur, der Citronensäurekonzentration in der wässerigen Lösung, der Konzentration und der Zugabegeschwindigkeit des Fällungsmittels usw. sowie aufgrund der Kenntnis der Begleitstoffe die optimalen Werte zu ermitteln. Da in der betrieblichen Praxis im allgemeinen über längere Zeiträume konstante Verhältnisse herrschen, brauchen die für den konkreten Fall günstigsten Parameter somit nur einmal bestimmt zu werden.

Die Erfindung wird in den folgenden Beispielen noch näher erläutert.

Nach sämtlichen Beispielen wurde eine aus der Vergärung von Rübenmelasse gewonnene Citronensäurefermentationslösung mit einem Gehalt von 95 g Citronensäure-Monohydrat/l eingesetzt, der End-pH-Wert der wässerigen Lösung wurde jeweils auf 5,5 eingestellt, die Temperatur der Suspension bei Zugabeende der restlichen Menge an Fällungsmittel wurde auf 90°C gehalten und das gefällte und abfiltrierte Citrat

wurde immer mit derselben Menge an Waschwasser gewaschen.

Zur objektiven Beurteilung der Reinheit des jeweils erhaltenen Tricalciumcitrat-Tetrahydrates wurde wie folgt vorgegangen:

0,25 g Calciumcitrat (feucht vom Filter) wurden in 10 ml konz. Schwefelsäure gelöst, die Lösung wurde 10 min lang im kochenden Wasserbad erhitzt und deren Extinktion in einer 1 cm-Küvette bei einer Wellenlänge von 420 nm gegen Schwefelsäure gemessen. Der Extinktionswert (im folgenden als E(RCS) bezeichnet) ist direkt proportional dem Verunreinigungsgrad des Calciumcitrates.

Diese Untersuchungsmethode entspricht grundsätzlich der Bestimmung der "Readily Carbonisable Substances" gemäß den verschiedenen Arzneibüchern (BP 68 oder BP 73), wonach ein bestimmter - nur durch Vergleich mit Standardlösungen ermittelter - Verfärbungsgrad bei in den Handel gebrachter Citronensäure nicht überschritten werden darf. Die Verfärbung der Schwefelsäurelösung wird durch die als Verunreinigungen in der Citronensäure oder im Citrat enthaltenen Begleitstoffe bewirkt.

Die Extinktionsmessung bei 420 nm in einem Photometer wurde als die bei weitem objektivere Methode gewählt.

**Beispiel 1**

1 l Fermentationslösung wurde in einem Becherglas auf 90°C erhitzt und mit der kontinuierlichen Zufuhr von Kalkmilch (Gehalt: 180 g CaO/l) mittels Dosierpumpe begonnen. Die Zugabegeschwindigkeit wurde auf 700 ml/h eingestellt. Gerührt wurde mit einem üblichen Propellerrührer, wobei auf die sofortige Verteilung der Kalkmilch in der Lösung geachtet wurde. Ab einem pH-Wert von 3,0, welcher nach Zugabe von etwa 58 ml Kalkmilch erreicht war, wurde die Zugabegeschwindigkeit der Kalkmilch auf 200 ml/h vermindert, die Lösung wurde auf knapp unterhalb ihres Siedepunktes erhitzt und laufend beobachtet. Bei pH 3,2 (etwa 75 ml Kalkmilch) trat die erste Trübung durch gebildetes Tricalciumcitrat-Tetrahydrat auf, worauf die Zufuhr der Kalkmilch sofort unterbrochen wurde. Trotzdem entstand aus der Lösung fortwährend weiterer Niederschlag, welcher unter dem Mikroskop als eindeutig kristallin erkennbar war.

Nach ca. 5 bis 8 min war der Kristallisationsvorgang im wesentlichen beendet und es wurde weiter Kalkmilch mit einer zulaufgeschwindigkeit von 700 ml/h zugesetzt, bis der vorbestimmte End-pH-Wert etwa 27 bis 30 min nach Beginn der Kalkmilchzugabe erreicht war. Anschließend wurde noch 10 min lang bei 90°C gerührt, sodann das Calciumcitrat abfiltriert und gewaschen. Das Calciumcitrat war rein weiß und wies eine E(RCS) von 0,38 auf. Unter dem Mikroskop war dasselbe Erscheinungsbild der Kristalle zu erkennen wie das während der 5- bis 8-minütigen Wartezeit beobachtete.

## Beispiel 2

Es wurde analog zu Beispiel 1 vorgegangen, nur wurde statt der Kalkmilch eine Suspension von 320 g $CaCO_3$ in 1 l Wasser zudosiert. Dies bewirkte wegen der $CO_2$-Entwicklung ein starkes Schäumen der Lösung, welches nur durch laufende Zugabe von Antischaummittel (Siliconöl) und stark verlangsamte Zugabegeschwindigkeit der ersten etwa 58 ml des Fällungsmittels (350 ml/h) unter Kontrolle gehalten werden konnte. Nach 5-minütiger Drosselung der Zugabegeschwindigkeit auf 200 ml/h trat wie im Beispiel 1 bei pH 3,2 die erste Trübung auf, woraufhin ebenfalls 5 bis 8 min lang die Zufuhr der Calciumcarbonat-Suspension unterbrochen wurde. Danach wurde die Fällung wieder mit entsprechend geringer Zugabegeschwindigkeit von 350 ml/h beendet und wie im vorangehenden Beispiel weitere 10 min lang bei 90° C gerührt.

Infolge der starken Schaumentwicklung und der dadurch bedingten langsameren Fällungsmittelzugabe dauerte der gesamte Fällungsvorgang 53 bis 56 min. Das filtrierte und gewaschene Calciumcitrat zeigte ein ähnliches mikroskopisches Erscheinungsbild wie das nach Beispiel 1 erhaltene; die E(RCS) betrug 0,42.

Die Wahl des Fällungsmittels hat somit keinen Einfluß auf die Qualität des entstandenen Produktes, es muß aber bei Verwendung von Calciumcarbonat eine längere Dauer des Verfahrens in Kauf genommen werden; außerdem ist die Gefahr des Übertretens von Schaum gegeben.

## Beispiel 3

Anstelle des in den Beispielen 1 und 2 verwendeten Propellerrührwerkes wurde ein Vibrationsmischer eingesetzt. Die Mischplatte war bei diesem Gerät mittels einer Hohlwelle mit dem eigentlichen Schwingungserzeuger verbunden. Das Fällungsmittel (Kalkmilch) wurde durch diese Hohlwelle in die Fermentationsflüssigkeit eingebracht. Da das Fällungsmittel direkt unterhalb der vibrierenden Platte aus der Hohlwelle austrat, wurde es augenblicklich in der gesamten Flüssigkeit feinst verteilt.

Wie in den vorhergehenden Beispielen wurde die Fermentationslösung auf 90° C erhitzt und mit der kontinuierlichen Zugabe der Kalkmilch mit einer Geschwindigkeit von 1200 ml/h begonnen. Ab pH 3,0 nach ca. 3 min wurde die Zufuhrgeschwindigkeit während weiterer 3 min auf 300 ml/h verlangsamt, wonach bei pH 3,2 die ersten Calciumcitratkristalle entstanden, worauf die Fällungsmittelzufuhr wieder 5 bis 8 min lang unterbrochen wurde.

Wie in den Beispielen 1 und 2 entstanden während dieser Zeitspanne laufend Tricalciumcitratkristalle aus der Lösung. Anschließend wurde die Fällung mit einer Zugabegeschwindigkeit von 1200 ml/h zu Ende geführt, 10 min lang bei 90° C gemischt, das Tricalciumcitrat-Tetrahydrat abfiltriert und gewaschen. Das Produkt war reinweiß und wies eine E(RCS) von 0,35 auf.

Als Folge der besonders effektiven Durchmischung der Flüssigkeit und der sofortigen Verteilung des Fällungsmittels mit Hilfe des Vibrationsmischers war es möglich, die Zugabegeschwindigkeit der Kalkmilch im Vergleich zu Beispiel 1 zu erhöhen, sodaß der Fällungsvorgang einschließlich der zehnminütigen Nachmischzeit insgesamt zur 28 bis 31 min lang dauerte.

Dies bedeutet, daß durch Einsatz eines Vibrationsmischers ein gut waschbares, kristallines Produkt ohne Zeitverzug im Vergleich zu einer üblichen Fällung, bei der die Gesamtmenge des Fällungsmittels mit konstanter Zulaufgeschwindigkeit zugesetzt wird, erhalten werden konnte.

## Beispiel 4

Bei sonst gleicher Arbeitsweise wie in Beispiel 3 beschrieben, wurde mit der Calciumcarbonat-Suspension gemäß Beispiel 2 gefällt. Es zeigte sich, daß die Schaumbildung wesentlich weniger intensiv war, wodurch die Zugabegeschwindigkeit der Suspension entsprechend erhöht werden konnte (4 min lang 900 ml/h, anschließend 3 min lang 300 ml/h, 5- bis 8-minütige Unterbrechung, restliche Menge wieder mit 900 ml/h).

Das erhaltene Tricalciumcitrat-Tetrahydrat war wieder hochrein und deutlich kristallin. Es wies eine E(RCS) von 0,34 auf und hatte damit die gleiche gute Qualität wie das nach Beispiel 3 erhaltene Produkt.

Wegen der im Vergleich zu Beispiel 2 zwar unterdrückten, aber naturgemäß nicht ganz vermeidbaren Schaumentwicklung dauerte der gesamte Fällungsvorgang 31 bis 34 min lang. Dies bedeutet jedoch keine wesentliche Verlängerung der Fällungsdauer im Vergleich zur der bei Verwendung von Kalkmilch resultierenden.

## Vergleichsbeispiel

Es wurde wie in Beispiel 1 vorgegangen, die Geschwindigkeit der Zugabe von Kalkmilch (700 ml/h) wurde jedoch nicht vermindert. Bei einem pH-Wert von 3,4 begann plötzlich Tricalciumcitrat-Tetrahydrat auszufallen, die Kalkmilchzufuhr wurde mit konstanter Geschwindigkeit ohne Unterbrechung fortgesetzt. Die Zugabe der gesamten, zur Bildung von Tricalciumcitrat benötigten Menge (theoretisch ca. 211 ml) an Kalkmilch dauerte etwa 18 min lang, hernach wurde noch 10 min lang bei 90° C gerührt und anschließend das gebildete Calciumcitrat abfiltriert und gewaschen. Es wies trotz Waschung eine deutlich braune Verfärbung auf, die E(RCS) wurde mit 0,96 festgestellt. Bei Betrachtung des Produktes unter dem Mikroskop konnten relativ große, dunkle Agglomerate von amorphem Tricalciumcitrat-Tetrahydrat beobachtet werden.

Es ist ersichtlich, daß das erfindungsgemäß hergestellte Citrat einen bei weitem höheren Reinheitsgrad aufweist.

**Patentansprüche**

1. Verfahren zur Isolierung von hochreinem kristallinem Tricalciumcitrat-Tetrahydrat durch Zusatz eines calciumhältigen Fällungsmittels zu citronensäurehältigen wässerigen Lösungen bei erhöhter Temperatur, dadurch gekennzeichnet, daß nach Zugabe von etwa 25 bis 30 % der zur Bildung von Tricalciumcitrat benötigten, stöchiometrisch errechneten Menge an calciumhältigem Fällungsmittel zu der wässerigen Lösung die weitere Zugabe mit verminderter Geschwindigkeit bis zum ersten Auftreten einer Trübung durch kristallin ausfallendes Tricalciumcitrat-Tetrahydrat fortgesetzt, dann so lange unterbrochen wird, bis der Kristallisationsvorgang im wesentlichen beendet ist und nach Ablauf dieser Zeitspanne die restliche Menge des calciumhältigen Fällungsmittels zugegeben wird.

2. Verfahren nach Anspruch 1, dadurch gekennzeichnet, daß die Zugabe des calciumhältigen Fällungsmittels ab einem pH-Wert der wässerigen Lösung von 3,0 mit verminderter Geschwindigkeit erfolgt.

3. Verfahren nach einem der Ansprüche 1 und 2, dadurch gekennzeichnet, daß die Zugabe des calciumhältigen Fällungsmittels beim ersten Auftreten einer Trübung 1 bis 30 min lang, vorzugsweise 5 bis 10 min lang, unterbrochen wird.

4. Verfahren nach einem der Ansprüche 1 bis 3, dadurch gekennzeichnet, daß von der restlichen Menge des calciumhältigen Fällungsmittels so viel zugesetzt wird, daß sich in der wässerigen Lösung ein pH-Wert von 5,5 einstellt.

5. Verfahren nach einem der Ansprüche 1 bis 4, dadurch gekennzeichnet, daß mit der Zugabe der ersten etwa 25 bis 30 % der Gesamtmenge an calciumhältigem Fällungsmittel bei einer Temperatur der wässerigen Lösung von 60 bis 90°C begonnen wird und die Temperatur der Lösung bei Zugabeende der restlichen Menge an calciumhältigem Fällungsmittel auf etwa 90°C bis 100°C eingestellt wird.

6. Verfahren nach einem der Ansprüche 1 bis 5, dadurch gekennzeichnet, daß die Zugabe der ersten etwa 25 bis 30 % sowie der restlichen Menge an calciumhältigem Fällungsmittel unter intensiver Durchmischung der Lösung mit hoher Geschwindigkeit - jedoch ohne lokale Überkonzentration von calciumhältigem Fällungsmittel in der Lösung - vorgenommen wird.

7. Verfahren nach einem der Ansprüche 1 bis 6, dadurch gekennzeichnet, daß die Lösung während der Zugabe des calciumhältigen Fällungsmittels mit einem eine Hohlwelle aufweisenden Vibrationsmischer intensiv durchmischt wird, wobei das calciumhältige Fällungsmittel durch die Hohlwelle zugegeben wird.

8. Verfahren nach Anspruch 7, dadurch gekennzeichnet, daß als calciumhältiges Fällungsmittel Calciumcarbonat zugegeben wird.

**Claims**

1. Method of isolating highly pure crystalline tricalciumcitrate-tetrahydrate by addition of a calcium-containing precipitant to citric-acid-containing aqueous solutions at elevated temperature, which method is characterised in that, after addition of about 25 to 30 % of the stoichiometrically calculated amount of calcium-containing precipitant, to the aqueous solution, required for the formation of tricalcium-citrate, the further addition is continued at a reduced rate until the first appearance of cloudiness due to tricalcium-citrate-tetrahydrate precipitating by crystallisation, then is interrupted as long as crystallisation is substantially completed and the remaining portion of the calcium-containing precipitant is added upon expiration of that period of time.

2. Method according to claim 1, characterized in that the addition of the calcium-containing precipitant is effected at a reduced rate starting with a pH of the aqueous solution of 3.0.

3. Method according to one of claims 1 and 2, characterised in that the addition of the calcium-containing precipitant is interrupted for 1 to 30 minutes, preferably 5 to 10 minutes, upon the first appearance of cloudiness.

4. Method according to one of claims 1 to 3, characterised in that of the remaining portion of the calcium-containing precipitant so much is added that a pH of 5.5 is reached in the aqueous solution.

5. Method according to one of claims 1 to 4, characterised in that the addition of the first approximately 25 to 30 % of the total amount of calcium-containing precipitant is started at a temperature of the aqueous solution of from 60 to 90°C, and the temperature of the solution, at the end of the addition of the remaining portion of calcium-containing precipitant, is adjusted to about 90°C to 100°C.

6. Method according to one of claims 1 to 5, characterised in that the addition of the first approximately 25 to 30 % as well as of the remaining portion of said calcium-containing precipitant is effected under intensive mixing of the solution at a high speed - yet without local superconcentration of the calcium-containing precipitant in the solution.

7. Method according to one of claims 1 to 6, characterised in that the solution is intensively mixed during the addition of the calcium-containing precipitant by a vibration mixer provided with a hollow shaft, the calcium-containing precipitant being supplied through the hollow shaft.

8. Method according to claim 7, characterised in that calcium carbonate is supplied as the calcium-containing precipitant.

**Revendications**

1. Procédé pour isoler du citrate tricalcique tétrahydraté cristallisé à haute pureté par addition d'un agent précipitant contenant du calcium à des solutions aqueuses contenant de l'acide citrique à chaud, caractérisé en ce que, après addition d'environ 25 à 30 % de la quantité d'agent précipitant contenant du calcium stoechiométrique calculée nécessaire pour la formation du citrate tricalcique à la solution aqueuse, on poursuit l'addition à une vitesse plus faible jusqu'à la première apparition d'un trouble dû à la formation de citrate tricalcique tétrahydraté cristallisé, puis on interrompt jusqu'à ce que le processus de cristallisation soit essentiellement terminé et, après cette période d'arrêt, on ajoute le restant de l'agent précipitant contenant du calcium.

2. Procédé selon la revendication 1, caractérisé en ce que l'addition de l'agent précipitant contenant du calcium est ralentie à partir du moment où le pH de la solution aqueuse est de 3,0.

3. Procédé selon l'une des revendications 1 et 2, caractérisé en ce que l'addition de l'agent précipitant contenant du calcium est interrompue à la première apparition d'un trouble pendant une durée de 1 à 30 min, de préférence de 5 à 10 min.

4. Procédé selon l'une des revendications 1 à 3, caractérisé en ce que, sur le restant de l'agent précipitant contenant du calcium, on ajoute une quantité telle que la solution aqueuse soit à un pH de 5,5.

5. Procédé selon l'une des revendications 1 à 4, caractérisé en ce que l'on commence l'addition de la première portion d'environ 25 à 30 % de la quantité totale d'agent précipitant contenant du calcium à une température de la solution aqueuse qui est de 60 à 90°C et on règle à un niveau de 90 à 100°C la température de la solution à la fin de l'addition du restant de l'agent précipitant contenant du calcium.

6. Procédé selon l'une des revendications 1 à 5, caractérisé en ce que l'addition de la première portion d'environ 25 à 30 % et l'addition du restant d'agent précipitant contenant du calcium sont effectuées sous mélange intensif de la solution à haute vitesse mais sans surconcentration locale de l'agent précipitant contenant du calcium dans la solution.

7. Procédé selon l'une des revendications 1 à 6, caractérisé en ce que, pendant l'addition de l'agent précipitant contenant du calcium, la solution est soumise à mélange intensif à l'aide d'un mélangeur à vibrations ayant un axe creux au travers duquel l'agent précipitant contenant du calcium est introduit.

8. Procédé selon la revendication 7, caractérisé en ce que l'agent précipitant contenant du calcium consiste en carbonate de calcium.